# EUROPEAN PATENT SPECIFICATION

(11) **EP 4 125 675 B1**
(45) Date of publication and mention of the grant of the patent: **08.10.2025**
(21) Application number: 21717688.2
(22) Date of filing: 16.03.2021
(51) Int. Cl.: A61B 34/20, A61B 90/00

(54) **SYSTEMS FOR REGISTERING AN INSTRUMENT TO AN IMAGE USING POINT CLOUD DATA AND ENDOSCOPIC IMAGE DATA**
SYSTEME ZUR REGISTRIERUNG EINES INSTRUMENTS AUF EINEM BILD UNTER VERWENDUNG VON PUNKTWOLKENDATEN UND ENDOSKOPISCHEN BILDDATEN
SYSTÈMES POUR ENREGISTRER UN INSTRUMENT SUR UNE IMAGE À L'AIDE DE DONNÉES DE NUAGE DE POINTS ET DE DONNÉES D'IMAGE ENDOSCOPIQUE

(30) Priority: 24.03.2020 US 202062994205 P
(43) Date of publication of application: 08.02.2023
(60) Divisional application: 25199872.0
(73) Proprietor: Intuitive Surgical Operations, Inc., Sunnyvale, CA 94086 (US)
(72) Inventor: SOPER, Timothy D., Sunnyvale, California 94086 (US); ZHANG, Hui, Sunnyvale, California 94086 (US)
(74) Representative: J A Kemp LLP
(86) International application number: PCT/US2021/022628
(87) International publication number: WO 2021/194803

(56) References cited:
- WO-A1-2019/245818
- US-A1- 2009 227 861
- US-A1- 2012 289 777
- US-A1- 2019 388 005

## Description

### CROSS-REFERENCE TO RELATED APPLICATION(S)

This application claims the benefit of U.S. Provisional Application No. 62/994,205, filed March 24, 2020.

### TECHNICAL FIELD

The present disclosure is directed to systems, methods, and computer program products for registering an instrument and image frames of reference by combining point cloud data and endoscopic image data.

### BACKGROUND

Minimally invasive medical techniques are intended to reduce the amount of tissue that is damaged during medical procedures, thereby reducing patient recovery time, discomfort, and harmful side effects. Such minimally invasive techniques may be performed through natural orifices in a patient anatomy or through one or more surgical incisions. Through these natural orifices or incisions, an operator may insert minimally invasive medical tools to reach a target tissue location. Minimally invasive medical tools include instruments such as therapeutic, diagnostic, biopsy, and surgical instruments. Medical tools may be inserted into anatomic passageways and navigated toward a region of interest within a patient anatomy. Navigation may be assisted using images of the anatomic passageways. Improved systems and methods are needed to accurately perform registrations between medical tools and images of the anatomic passageways.

US 2012/289777 A1 discloses a medical system for providing navigation assistance to a surgeon so that the surgeon may navigate a flexible medical device through linked passages of an anatomical structure to a target in or adjacent to the anatomical structure. As the medical device moves through the linked passages, images are captured by an image capturing element at its distal end and pose and shape information for the medical device are received from sensors disposed in the medical device. A 4-D computer model of the anatomical structure is registered to the medical device using one or both of 4-D shape registration and virtual camera registration so that the captured image and a virtual image generated from the perspective of a virtual camera are registered to each other and displayed while providing an indication of a navigational path to the target.

US 2019/245818 A1 discloses a system including one or more processors configured to read instructions to cause the system to perform operations including accessing a set of model points of a model of an anatomic structure of a patient. The model points are associated with a model space. The operations further include collecting a set of measured points of the anatomic structure of the patient, the measured points being associated with a patient space, determining a set of matches between the set of model points and the set of measured points, determining a first plurality of weights for the set of matches, and registering the set of model points to the set of measured points based on the first plurality of weights to generate a first registration.

### SUMMARY

Disclosed herein are devices, systems, methods, and computer program products for combining positional sensor data (e.g., shape and/or electro-magnetic sensor data) and endoscopic image data (e.g., video data, still images, etc.) to improve registration between (i) real patient anatomy (e.g., airways of lungs of a patient) within an anatomic region of a patient navigated by a medical instrument system as part of an image-guided medical procedure and (ii) an image of the anatomic region (e.g., generated from preoperative and/or intraoperative imaging).

The scope of the invention is defined in the appended claims. Any "aspect", "example" and "embodiment" of the description not falling within the scope of the claims does not form part of the invention and is provided for illustrative purposes only.

It is to be understood that both the foregoing general description and the following details description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure. The drawings should not be taken to limit the disclosure to the specific embodiments depicted, but are for explanation and understanding only.
FIG. 1 is a schematic representation of a robotic or teleoperated medical system configured in accordance with various embodiments of the present technology.
FIG. 2 is a schematic representation of a manipulator assembly, a medical instrument system, and an imaging system configured in accordance with various embodiments of the present technology.
FIG. 3 is a schematic representation of a portion of the medical instrument system of FIG. 2 extended within an anatomic region of a patient in accordance with various embodiments of the present technology.
FIG. 4 illustrates a plurality of coordinate points forming a point cloud representing a shape of the portion of the medical instrument system of FIG. 3 configured in accordance with various embodiments of the present technology.
FIG. 5 illustrates a real navigational image of real patient anatomy from a viewpoint of the portion of the medical instrument system of FIG. 3 extended within the anatomic region of FIG. 3 in accordance with various embodiments of the present technology.
FIG. 6 illustrates an intraoperative image of a portion of the anatomic region of FIG. 3 while the portion of the medical instrument system of FIG. 3 is extended within the anatomic region in accordance with various embodiments of the present technology.
FIG. 7 is a schematic representation of a display of a display system displaying a composite virtual navigational image in which the medical instrument system of FIGS. 2 and 3 is registered to an anatomic model of the anatomic region of FIG. 3, a virtual navigational image of virtual patient anatomy, and a real navigational image of real patient anatomy within the anatomic region in accordance with various embodiments of the present technology.
FIG. 8 is a flow diagram illustrating a method for registering an image of an anatomic region of a patient with a point cloud of coordinate points using endoscopic image data in accordance with various embodiments of the present technology.
FIG. 9 is schematic representation of an anatomic region of a patient and real navigational images of real patient anatomy within the anatomic region from a viewpoint of a medical instrument system extended various depths within the anatomic region in accordance with various embodiments of the present technology.
FIG. 10 illustrates a real navigational image of patient anatomy from a viewpoint of a medical instrument system extended within an anatomic region of a patient in accordance with various embodiments of the present technology.
FIGS. 11A and 11B illustrate virtual navigational images depicting virtual patient anatomy of the anatomic region of FIG. 10 from a viewpoint of a medical instrument system at a location within an anatomic model of the anatomic region corresponding to a location of the medical instrument system extended within the anatomic region.
FIG. 12 illustrates a virtual navigational image depicting virtual patient anatomy of an anatomic region from a viewpoint of a medical instrument system at a location within an anatomic model of the anatomic region corresponding to a location of the medical instrument system extended within the anatomic region.
FIGS. 13A-13C illustrate real navigational images of patient anatomy within the anatomic region of FIG. 11 from a viewpoint of the medical instrument system extended at various depths within the anatomic region.

### DETAILED DESCRIPTION

The present disclosure is directed to devices, systems, methods, and computer program products for combining positional sensor data (e.g., shape and/or electro-magnetic sensor data) and endoscopic image data (e.g., video data, still images, etc.) to improve registration between (i) real patient anatomy (e.g., airways of lungs of a patient) within an anatomic region of a patient navigated by a medical instrument system as part of an image-guided medical procedure and (ii) an image of the anatomic region (e.g., generated from preoperative and/or intraoperative imaging). When adequately registered, the tracked position of the medical instrument system within the anatomic region can be mapped to a correct position within an anatomic model of the anatomic region for use in guiding navigation of the medical instrument system throughout the anatomic region and/or for use in guiding interaction with subsurface structures within and/or near the anatomic region (e.g., for use in guiding a biopsy and/or treatment of nodules of the lungs). In particular, the present technology provides visual guidance in the form of a virtual navigational (e.g., fly-through) images from the viewpoint of the medical instrument system within the anatomic region that are generated within the anatomic model at the location of the medical instrument system following registration.

In some embodiments, the steps of registering the real patient anatomy to the anatomic model include: (a) navigating a medical instrument system throughout an anatomic region of a patient, (b) generating a point cloud of coordinate points representing locations visited by (e.g., a distal portion of) the medical instrument system, and (c) registering the point cloud (using an iterative closest point algorithm) to an image (e.g., a segmented CT image) of the anatomic region. In these and other embodiments, the present technology captures endoscopic image data (e.g., video data, still images, etc.) including a real navigational image of real patient anatomy within the anatomic region using an endoscope or other image capture device mounted to the distal portion (or another suitable location) of the medical instrument system. In these and still other embodiments, the present technology computes a virtual navigational image based, at least in part, on the registration. The virtual navigational image depicts virtual patient anatomy of the anatomic region from the perspective of the distal portion (or another suitable location) of the medical instrument system.

In some embodiments, the extent to which the virtual navigational image of virtual patient anatomy matches the real navigational image of real patient anatomy of the anatomic region provides an indication of how well the point cloud of coordinate points registers with the image (e.g., with the segmented CT image) of the anatomic region. The present technology therefore leverages information provided by both the real and virtual navigational images to improve the registration of the point cloud generated from data captured by the medical instrument system with the preoperative and/or intraoperative image of the anatomic region. In the context of biopsy medical procedures, the present technology thereby increases localization accuracy of regions of interest (e.g., tumor position estimations), which increases the probability of successfully navigating an anatomic region of a patient and the probability of effectively diagnosing and treating disease (e.g., of effectively biopsying or ablating small lung tumors).

### A. Embodiments of Robotic or Teleoperated Medical Systems and Associated Devices, Systems, and Methods

### 1. Robotic or Teleoperated Medical Systems and Associated Devices and Systems

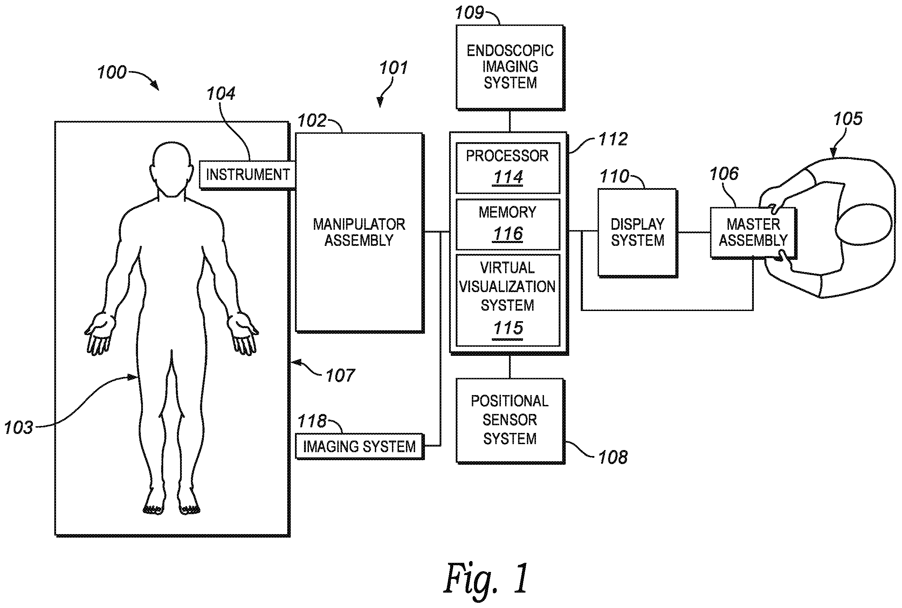

FIG. 1 is a schematic representation of a robotic or teleoperated medical system 100 ("medical system 100") configured in accordance with various embodiments of the present technology. As shown, the medical system 100 includes a manipulator assembly 102, a medical instrument system 104, a master assembly 106, and a control system 112. The manipulator assembly 102 supports the medical instrument system 104 and drives the medical instrument system 104 at the direction of the master assembly 106 and/or the control system 112 to perform various medical procedures on a patient 103 positioned on a table 107 in a surgical environment 101. In this regard, the master assembly 106 generally includes one or more control devices that can be operated by an operator 105 (e.g., a physician) to control the manipulator assembly 102. Additionally, or alternatively, the control system 112 includes a computer processor 114 and at least one memory 116 for effecting control between the medical instrument system 104, the master assembly 106, and/or other components of the medical system 100. The control system 112 can also include programmed instructions (e.g., a non-transitory computer-readable medium storing the instructions) to implement any one or more of the methods described herein, including instructions for providing information to a display system 110 and/or processing data for registration of the medical instrument system 104 with an anatomic model of the patient 103 (as described in greater detail below). The manipulator assembly 102 can be a teleoperated, a non-teleoperated, or a hybrid teleoperated and non-teleoperated assembly. Thus, all or a portion of the master assembly 106 and/or all or a portion of the control system 112 can be positioned inside or outside of the surgical environment 101.

To aid the operator 105 in controlling the manipulator assembly 102 and/or the medical instrument system 104 during an image-guided medical procedure, the medical system 100 may further include a positional sensor system 108, an endoscopic imaging system 109, an imaging system 118, and/or a virtual visualization system 115. In some embodiments, the positional sensor system 108 includes a location sensor system (e.g., an electromagnetic (EM) sensor system) and/or a shape sensor system for capturing positional sensor data (e.g., position, orientation, speed, velocity, pose, shape, etc.) of the medical instrument system 104. In these and other embodiments, the endoscopic imaging system 109 includes one or more image capture devices (not shown) that record endoscopic image data that includes concurrent or real-time images (e.g., video, still images, etc.) of patient anatomy. Images captured by the endoscopic imaging system 109 may be, for example, two or three-dimensional images of patient anatomy captured by an image capture device positioned within the patient 103, and are referred to hereinafter as "real navigational images."

In some embodiments, the medical instrument system 104 may include components of the positional sensor system 108 and/or components of the endoscopic imaging system 109. For example, components of the positional sensor system 108 and/or components of the endoscopic imaging system 109 can be integrally or removably coupled to the medical instrument system 104. Additionally, or alternatively, the endoscopic imaging system 109 can include a separate endoscope (not shown) attached to a separate manipulator assembly (not shown) that can be used in conjunction with the medical instrument system 104 to image patient anatomy. The positional sensor system 108 and/or the endoscopic imaging system 109 may be implemented as hardware, firmware, software, or a combination thereof that interact with or are otherwise executed by one or more computer processors, such as the computer processor(s) 114 of the control system 112.

The imaging system 118 of the medical system 100 may be arranged in the surgical environment 101 near the patient 103 to obtain real-time and/or near real-time images of the patient 103 before, during, and/or after a medical procedure. In some embodiments, the imaging system 118 includes a mobile C-arm cone-beam computerized tomography (CT) imaging system for generating three-dimensional images. For example, the imaging system 118 can include a DynaCT imaging system from Siemens Corporation, or another suitable imaging system. In these and other embodiments, the imaging system 118 can include other imaging technologies, including magnetic resonance imaging (MRI), fluoroscopy, thermography, ultrasound, optical coherence tomography (OCT), thermal imaging, impedance imaging, laser imaging, nanotube X-ray imaging, and/or the like.

The virtual visualization system 115 of the control system 112 provides navigation and/or anatomy-interaction assistance to the operator 105 when controlling the medical instrument system 104 during an image-guided medical procedure. As described in greater detail below, virtual navigation using the virtual visualization system 115 can be based, at least in part, upon reference to an acquired pre-operative or intra-operative dataset (e.g., based, at least in part, upon reference to data generated by the positional sensor system 108, the endoscopic imaging system 109, and/or the imaging system 118) of anatomic passageways of the patient 103. In some implementations, for example, the virtual visualization system 115 processes preoperative and/or intraoperative image data of an anatomic region of the patient 103 captured by the imaging system 118 to generate an anatomic model (not shown) of the anatomic region. The virtual visualization system 115 then registers the anatomic model to positional sensor data generated by the positional sensor system 108 and/or to endoscopic image data generated by the endoscopic imaging system 109 to (i) map the tracked position, orientation, pose, shape, and/or movement of the medical instrument system 104 within the anatomic region to a correct position within the anatomic model, and/or (ii) determine a virtual navigational image of virtual patient anatomy of the anatomic region from a viewpoint of the medical instrument system 104 at a location within the anatomic model corresponding to a location of the medical instrument system 104 within the patient 103.

The display system 110 can display various images or representations of patient anatomy and/or of the medical instrument system 104 that are generated by the positional sensor system 108, by the endoscopic imaging system 109, by the imaging system 118, and/or by the virtual visualization system 115. In some embodiments, the display system 110 and/or the master assembly 106 may be oriented so the operator 105 can control the manipulator assembly 102, the medical instrument system 104, the master assembly 106, and/or the control system 112 with the perception of telepresence.

As discussed above, the manipulator assembly 102 drives the medical instrument system 104 at the direction of the master assembly 106 and/or the control system 112. In this regard, the manipulator assembly 102 can include select degrees of freedom of motion that may be motorized and/or teleoperated and select degrees of freedom of motion that may be non-motorized and/or non-teleoperated. For example, the manipulator assembly 102 can include a plurality of actuators or motors (not shown) that drive inputs on the medical instrument system 104 in response to commands received from the control system 112. The actuators can include drive systems (not shown) that, when coupled to the medical instrument system 104, can advance the medical instrument system 104 into a naturally or surgically created anatomic orifice. Other drive systems may move a distal portion (not shown) of the medical instrument system 104 in multiple degrees of freedom, which may include three degrees of linear motion (e.g., linear motion along the X, Y, Z Cartesian axes) and three degrees of rotational motion (e.g., rotation about the X, Y, Z Cartesian axes). Additionally, or alternatively, the actuators can be used to actuate an articulable end effector of the medical instrument system 104 (e.g., for grasping tissue in the jaws of a biopsy device and/or the like).

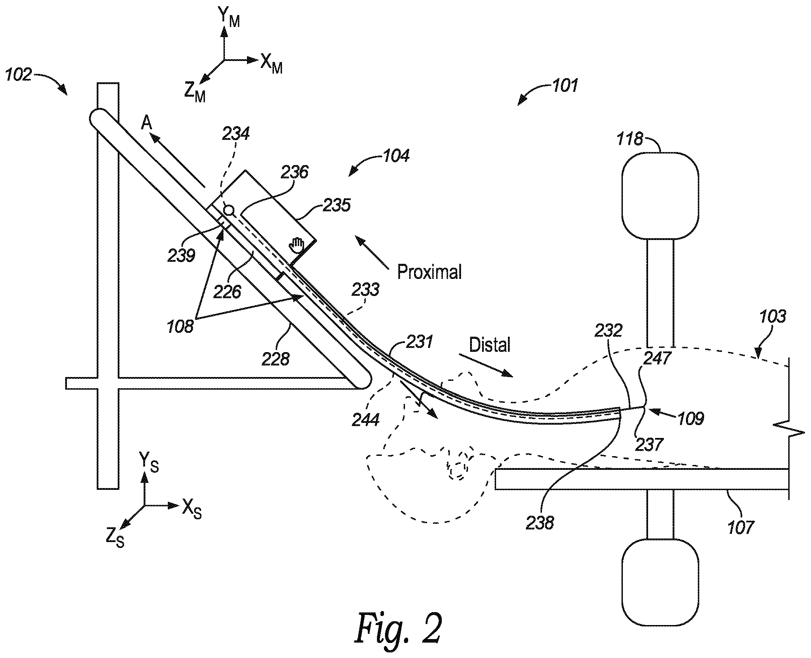

FIG. 2 is a schematic representation of the manipulator assembly 102, the medical instrument system 104, and the imaging system 118 of FIG. 1 within the surgical environment 101 and configured in accordance with various embodiments of the present technology. As shown in FIG. 2, the surgical environment 101 has a surgical frame of reference (X_{S}, Y_{S}, Z_{S}) in which the patient 103 is positioned on the table 107, and the medical instrument system 104 has a medical instrument frame of reference (X_{M}, Y_{M}, Z_{M}) within the surgical environment 101. During the medical procedure, the patient 103 may be stationary within the surgical environment 101 in the sense that gross patient movement can be limited by sedation, restraint, and/or other means. In these and other embodiments, cyclic anatomic motion of the patient 103, including respiration and cardiac motion, may continue unless the patient 103 is asked to hold his or her breath to temporarily suspend respiratory motion.

The manipulator assembly 102 includes an instrument carriage 226 mounted to an insertion stage 228. In the illustrated embodiment, the insertion stage 228 is linear, while in other embodiments, the insertion stage 228 is curved or has a combination of curved and linear sections. In some embodiments, the insertion stage 228 is fixed within the surgical environment 101. Alternatively, the insertion stage 228 can be movable within the surgical environment 101 but have a known location (e.g., via a tracking sensor (not shown) or other tracking device) within the surgical environment 101. In these alternatives, the medical instrument frame of reference (X_{M}, Y_{M}, Z_{M}) is fixed or otherwise known relative to the surgical frame of reference (X_{S}, Y_{S}, Z_{S}).

The medical instrument system 104 of FIG. 2 includes an elongate device 231, a medical instrument 232, an instrument body 235, at least a portion of the positional sensor system 108, and at least a portion of the endoscopic imaging system 109. In some embodiments, the elongate device 231 is a flexible catheter or other biomedical device that defines a channel or lumen 244. The channel 244 can be sized and shaped to receive the medical instrument 232 (e.g., via a proximal end 236 of the elongate device 231 and/or an instrument port (not shown)) and facilitate delivery of the medical instrument 232 to a distal portion 238 of the elongate device 231. The elongate device 231 is coupled to the instrument body 235, which in turn is coupled and fixed relative to the instrument carriage 226 of the manipulator assembly 102.

In operation, the manipulator assembly 102 can control insertion motion (e.g., proximal and/or distal motion along an axis A) of the elongate device 231 into the patient 103 via a natural or surgically created anatomic orifice of the patient 103 to facilitate navigation of the elongate device 231 through anatomic passageways of an anatomic region of the patient 103 and/or to facilitate delivery of a distal portion 238 of the elongate device 231 to or near a target location within the patient 103. For example, the instrument carriage 226 and/or the insertion stage 228 may include actuators (not shown), such as servomotors, that facilitate control over motion of the instrument carriage 226 along the insertion stage 228. Additionally, or alternatively, the manipulator assembly 102 in some embodiments can control motion of the distal portion 238 of the elongate device 231 in multiple directions, including yaw, pitch, and roll rotational directions (e.g., to navigate patient anatomy). To this end, the elongate device 231 may house or include cables, linkages, and/or other steering controls (not shown) that the manipulator assembly 102 can use to controllably bend the distal portion 238 of the elongate device 231. For example, the elongate device 231 can house at least four cables that can be used by the manipulator assembly 102 to provide (i) independent "up-down" steering to control a pitch of the distal portion 238 of the elongate device 231 and (ii) independent "left-right" steering of the elongate device 231 to control a yaw of the distal portion 238 of the elongate device 231.

The medical instrument 232 of the medical instrument system 104 can be used for medical procedures, such as for survey of anatomic passageways, surgery, biopsy, ablation, illumination, irrigation, and/or suction. Thus, the medical instrument 232 can include image capture probes, biopsy instruments, laser ablation fibers, and/or other surgical, diagnostic, and/or therapeutic tools. For example, the medical instrument 232 includes an endoscope or other biomedical device having one or more image capture devices 247 positioned at a distal portion 237 of the medical instrument 232. The image capture device 247 can capture one or more real navigational images or video (e.g., a sequence of one or more real navigational image frames) of anatomic passageways and/or other real patient anatomy while the medical instrument 232 is within an anatomic region of the patient 103.

As discussed above, the medical instrument 232 can be deployed into and/or be delivered to a target location within the patient 103 via the channel 244 defined by the elongate device 231. As the medical instrument 232 includes an endoscope or other biomedical device having an image capture device 247 at its distal portion 237, the image capture device 247 can be advanced to the distal portion 238 of the elongate device 231 before, during, and/or after the manipulator assembly 102 navigates the distal portion 238 of the elongate device 231 to a target location within the patient 103. The medical instrument 232 can be used as a survey instrument to capture real navigational images of anatomic passageways and/or other real patient anatomy, and/or to aid an operator (not shown) to navigate the distal portion 238 of the elongate device 231 through anatomic passageways to the target location.

As another example, after the manipulator assembly 102 positions the distal portion 238 of the elongate device 231 proximate a target location within the patient 103, the medical instrument 232 can be advanced beyond the distal portion 238 of the elongate device 231 to perform a medical procedure at the target location. Continuing with this example, after all or a portion of the medical procedure at the target location is complete, the medical instrument 232 can be retracted back into the elongate device 231 and, additionally or alternatively, be removed from the proximal end 236 of the elongate device 231 or from another instrument port (not shown) along the elongate device 231.

As shown in FIG. 2, the positional sensor system 108 of the medical instrument system 104 includes a shape sensor 233 and a position measuring device 239. In these and other embodiments, the positional sensor system 108 can include other position sensors (e.g., accelerometers, rotary encoders, etc.) in addition to or in lieu of the shape sensor 233 and/or the position measuring device 239.

The shape sensor 233 of the positional sensor system 108 includes an optical fiber extending within and aligned with the elongate device 231. In one embodiment, the optical fiber of the shape sensor 233 has a diameter of approximately 200µm. In other embodiments, the diameter of the optical fiber may be larger or smaller. The optical fiber of the shape sensor 233 forms a fiber optic bend sensor that is used to determine a shape, orientation, and/or pose of the elongate device 231. In some embodiments, optical fibers having Fiber Bragg Gratings (FBGs) can be used to provide strain measurements in structures in one or more dimensions. Various systems and methods for monitoring the shape and relative position of an optical fiber in three dimensions are described in further detail in U.S. Patent Application Publication No. 2006/0013523 (filed July 13, 2005) (disclosing fiber optic position and shape sensing device and method relating thereto); U.S. Patent No. 7,781,724 (filed on September 26, 2006) (disclosing fiber-optic position and shape sensing device and method relating thereto); U.S. Patent No. 7,772541 (filed on March 12, 2008) (disclosing fiber-optic position and/or shape sensing based on Rayleigh scatter); and U.S. Patent No. 6,389,187 (filed on Jun. 17, 1998) (disclosing optical fiber bend sensors). In these and other embodiments, sensors of the present technology may employ other suitable strain sensing techniques, such as Rayleigh scattering, Raman scattering, Brillouin scattering, and Fluorescence scattering. In these and still other embodiments, the shape of the elongate device 231 may be determined using other techniques. For example, a history of the pose of the distal portion 238 of the elongate device 231 can be used to reconstruct the shape of elongate device 231 over an interval of time.

In some embodiments, the shape sensor 233 is fixed at a proximal point 234 on the instrument body 235 of the medical instrument system 104. In operation, for example, the shape sensor 233 measures a shape in the medical instrument reference frame (X_{M}, Y_{M}, Z_{M}) from the proximal point 234 to another point along the optical fiber, such as the distal portion 238 of the elongate device 231. The proximal point 234 of the shape sensor 233 may be movable along with instrument body 235 but the location of proximal point 234 may be known (e.g., via a tracking sensor (not shown) or other tracking device).

The position measuring device 239 of the positional sensor system 108 provides information about the position of the instrument body 235 as it moves along the insertion axis A on the insertion stage 228 of the manipulator assembly 102. In some embodiments, the position measuring device 239 includes resolvers, encoders, potentiometers, and/or other sensors that determine the rotation and/or orientation of actuators (not shown) controlling the motion of the instrument carriage 226 of the manipulator assembly 102 and, consequently, the motion of the instrument body 235 of the medical instrument system 104.

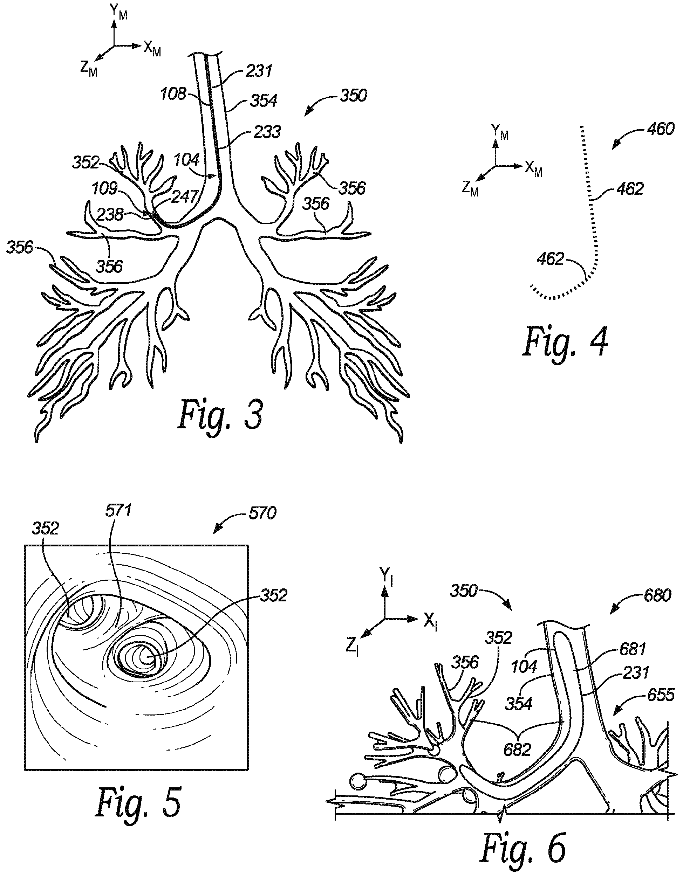

FIG. 3 is a schematic representation of a portion of the medical instrument system 104 of FIG. 2 extended within an anatomic region 350 (e.g., human lungs) of the patient 103 in accordance with various embodiments of the present technology. In particular, FIG. 3 illustrates the elongate device 231 of the medical instrument system 104 extending within branched anatomic passageways 352 of the anatomic region 350. The anatomic passageways 352 include a trachea 354 and a plurality of bronchial tubes 356.

As shown in FIG. 3, the elongate device 231 has a position, orientation, pose, and shape within the anatomic region 350, all or a portion of which (in addition to or in lieu of movement, such as speed or velocity) can be captured as positional sensor data by the positional sensor system 108 of FIGS. 1 and 2 (e.g., by the shape sensor 233 and/or the position measuring device 239 (FIG. 2)) to survey the anatomic passageways 352 of the anatomic region 350. In particular, the positional sensor system 108 can survey the anatomic passageways 352 by gathering positional sensor data of the medical instrument system 104 within the anatomic region 350 in the medical instrument frame of reference (X_{M}, Y_{M}, Z_{M}). The positional sensor data may at least in part be recorded as a set of two-dimensional or three-dimensional coordinate points. In the example of the anatomic region 350 being human lungs, the coordinate points may represent the locations of the distal portion 238 of the elongate device 231 and/or of other portions of the elongate device 231 while the elongate device 231 is advanced through the trachea 354 and the bronchial tubes 356. In these and other embodiments, the collection of coordinate points may represent the shape(s) of the elongate device 231 while the elongate device 231 is advanced through the anatomic region 350. In these and still other embodiments, the coordinate points may represent positional data of other portions (e.g., the medical instrument 232 (FIG. 2)) of the medical instrument system 104.

The coordinate points may together form a point cloud. For example, FIG. 4 illustrates a plurality of coordinate points 462 forming a point cloud 460 representing a shape of the elongate device 231 of FIG. 3 while the elongate device 231 is within the anatomic region 350 (FIG. 3) in accordance with various embodiments of the present technology. In particular, the point cloud 460 of FIG. 4 is generated from the union of all or a subset of the coordinate points 462 recorded by the positional sensor system 108 (FIG. 2) while the elongate device 231 is in the stationary position illustrated in FIG. 3.

In some embodiments, a point cloud (e.g., the point cloud 460) can include the union of all or a subset of coordinate points recorded by the positional sensor system 108 during an image capture period that spans multiple shapes, positions, orientations, and/or poses of the elongate device 231 within the anatomic region 350. In these embodiments, the point cloud can include coordinate points captured by the positional sensor system 108 that represent multiple shapes of the elongate device 231 while the elongate device 231 is advanced or moved through patient anatomy during the image capture period. Additionally, or alternatively, because the configuration, including shape and location, of the elongate device 231 within the patient 103 may change during the image capture period due to anatomical motion, the point cloud in some embodiments can comprise a plurality of coordinate points 462 captured by the positional sensor system 108 that represent the shapes of the elongate device 231 as the elongate device 231 passively moves within the patient 103. As described in greater detail below, a point cloud of coordinate points captured by the positional sensor system 108 can be registered to different models or datasets of patient anatomy.

Referring again to FIG. 2, the endoscopic imaging system 109 of the medical instrument system 104 includes one or more image capture devices 247 configured to capture one or more real navigational images of real patient anatomy (e.g., the anatomic passageways 352 of FIG. 3) while the elongate device 231 and/or the medical instrument 232 is within an anatomic region (e.g., the anatomic region 350 of FIG. 3) of the patient 103. The endoscopic imaging system 109 can include an image capture device 247 positioned at the distal portion 237 of the medical instrument 232. In these and other embodiments, the endoscopic imaging system 109 can include one or more image capture devices (not shown) positioned at other locations along the medical instrument 232 and/or along the elongate device 231 (e.g., at the distal portion 238 of the elongate device 231).

In the embodiment illustrated in FIG. 3, the image capture device 247 of the medical instrument 232 (FIG. 2) is advanced to and positioned at the distal portion 238 of the elongate device 231. In this embodiment, the image capture device 247 can survey the anatomic passageways 352 by capturing real navigational images of the anatomic passageways 352 while the elongate device 231 is navigated through the trachea 354 and the bronchial tubes 356 of the anatomic region 350.

FIG. 5 is an example of a real navigational image 570 (e.g., a still image, an image frame of a video, etc.) of patient anatomy of the anatomic region 350 of FIG. 3 (such as one of the anatomic passageways 352) captured via the image capture device 247 (FIG. 3). As shown, the real navigational image 570 shows a branching point or carina 571 of two anatomic passageways 352 within the anatomic region 350 from a viewpoint of the medical instrument 232 (FIG. 2). In this example, because the image capture device 247 is positioned at the distal portions 237 and 238 of the medical instrument 232 and the elongate device 231 (FIG. 3), respectively, the viewpoint of the real navigational image 570 is from the distal portion 237 of the medical instrument 232 such that the medical instrument 232 and the elongate device 231 are not visible within the real navigational image 570. In other embodiments, the image capture device 247 can be positioned at another location along the medical instrument 232 and/or along the elongate device 231 (FIGS. 2 and 3). In these embodiments, the endoscopic imaging system 109 (FIG. 2) can capture real navigational images from a corresponding viewpoint of the medical instrument 232 and/or of the elongate device 231. A portion of the medical instrument 232 and/or of the elongate device 231 may be visible within these real navigational images depending on the positions of the medical instrument 232 and the elongate device 231 relative to one another.

Referring again to FIG. 2, the real navigational images captured by the endoscopic imaging system 109 can facilitate navigation of the distal portion 238 of the elongate device 231 through patient anatomy (e.g., through the anatomic passageways 352 of FIG. 3) and/or delivery of the distal portion 238 of the elongate device 231 to a target location within the patient 103. In these and other embodiments, the real navigational images captured by the endoscopic imaging system 109 can facilitate (i) navigation of the distal portion 237 of the medical instrument 232 beyond the distal portion 238 of the elongate device 231, (ii) delivery of the distal portion 237 of the medical instrument 232 to a target location within the patient 103, and/or (iii) visualization of patient anatomy during a medical procedure. In some embodiments, each real navigational image captured by the endoscopic imaging system 109 can be associated with a time stamp and/or a position recorded in the medical instrument frame of reference (X_{M}, Y_{M}, Z_{M}). As described in greater detail below, the real navigational images captured by the endoscopic imaging system 109 can therefore be used to improve a registration between a point cloud of coordinate points (e.g., the point cloud 460 of FIG. 4) generated by the positional sensor system 108 and image data captured by the imaging system 118.

As shown in FIG. 2, the imaging system 118 is arranged near the patient 103 to obtain three-dimensional images of the patient 103 (e.g., of the anatomic region 350 of FIG. 3). In some embodiments, the imaging system 118 includes one or more imaging technologies, including CT, MRI, fluoroscopy, thermography, ultrasound, optical coherence tomography (OCT), thermal imaging, impedance imaging, laser imaging, nanotube X-ray imaging, and/or the like. The imaging system 118 is configured to generate image data of patient anatomy before, during, and/or after the elongate device 231 is extended within the patient 103. Thus, the imaging system 118 can be configured to capture preoperative, intraoperative, and/or postoperative three-dimensional images of patient anatomy. In these and other embodiments, the imaging system 118 may provide real-time or near real-time images of patient anatomy.

FIG. 6 illustrates an example of intraoperative image data 680 of a portion 655 of the anatomic region 350 of FIG. 3 captured during an image capture period by the imaging system 118 (FIG. 2) while the elongate device 231 of the medical instrument system 104 is extended within the anatomic region 350. As shown, the image data 680 includes graphical elements 681 representing the elongate device 231 and graphical elements 682 representing the anatomic passageways 352 of the anatomic region 350.

All or a portion of the graphical elements 681 and 682 of the image data 680 can be segmented and/or filtered to generate a virtual, three-dimensional model of the anatomic passageways 352 within the portion 655 of the anatomic region 350 (with or without the medical instrument system 104). In some embodiments, the graphical elements 681 and 682 can additionally or alternatively be segmented and/or filtered to generate an image point cloud (not shown) of the medical instrument system 104 based, at least in part, on images captured by the imaging system 118 (FIG. 2) while the medical instrument system 104 is within the anatomic region 350. During the segmentation process, pixels or voxels generated from the image data 680 may be partitioned into segments or elements or be tagged to indicate that they share certain characteristics or computed properties such as color, density, intensity, and texture. The segments or elements may then be converted to an anatomic model and/or to an image point cloud of the medical instrument system 104. Additionally, or alternatively, the segments or elements can be used to locate (e.g., calculate) and/or define a center line or other points running along the anatomic passageways 352. The generated anatomic model and/or the image point cloud may be two or three-dimensional and may be generated in an image reference frame (X_{I}, Y_{I}, Z_{I}).

As discussed above with respect to FIG. 1, the display system 110 (FIG. 1) of the medical system 100 (FIG. 1) can display various images or representations of patient anatomy and/or of the medical instrument system 104 based, at least in part, on data captured and/or generated by the positional sensor system 108, by the endoscopic imaging system 109, by the imaging system 118, and/or by the virtual visualization system 115. In various implementations, the images and/or representations can be utilized by the system to aid the operator 105 (FIG. 1) in conducting an image-guided medical procedure.

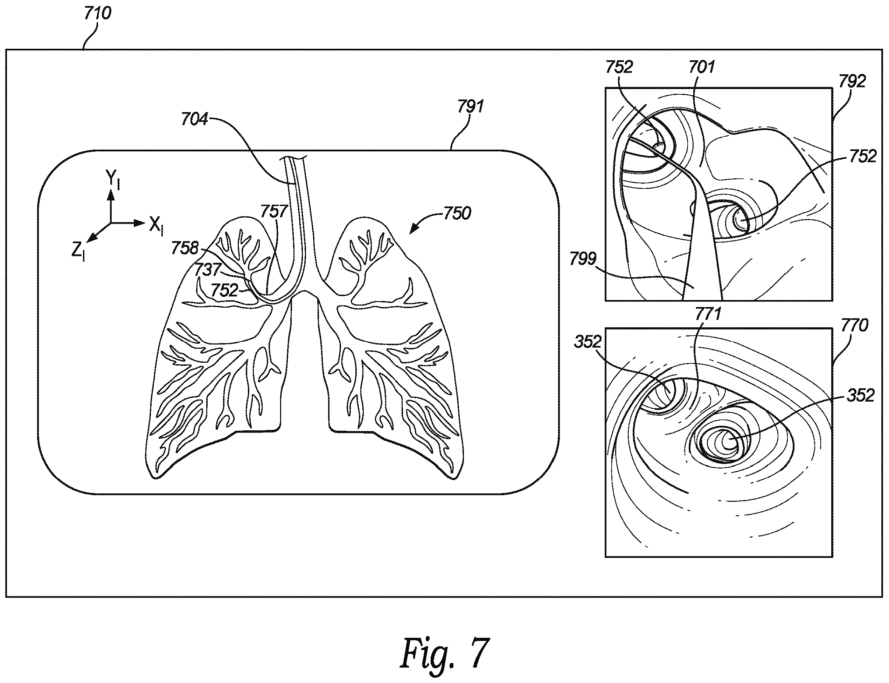

FIG. 7 is a schematic representation of an example display 710 produced by the display system 110 (FIG. 1) in accordance with various embodiments of the present technology. As shown, the display 710 includes a real navigational image 770, a composite virtual navigational image 791 (also referred to as a "composite virtual image 791"), and a virtual navigational image 792. The real navigational image 770 can be substantially the same as the real navigational image 570 of FIG. 5. Thus, for example, the real navigational image 770 can be captured by the endoscopic imaging system 109 (FIG. 2) and provided to the display system 110 (FIG. 1) to be presented on the display 710 in real-time or near real-time. In the illustrated embodiment, the real navigational image 770 illustrates real patient anatomy (e.g., a carina 771 marking a branching point of two anatomic passageways 352) from a viewpoint oriented distally away from the distal portion 237 of the medical instrument 232 (FIG. 2).

The composite virtual image 791 of FIG. 7 is displayed in the image reference frame (X_{I}, Y_{I}, Z_{I}) and includes an anatomic model 750 generated from image data of the anatomic region 350 of FIG. 3 captured by the imaging system 118 (FIG. 2). The anatomic model 750 is registered (i.e., dynamically referenced) with a point cloud of coordinate points (e.g., the point cloud 460 of FIG. 4) generated by the positional sensor system 108 (FIG. 2) to display a representation 704 within the anatomic model 750 of the tracked position, shape, pose, orientation, and/or movement of the medical instrument system 104 (e.g., of the elongate device 231 of FIG. 2) within the patient 103 (FIG. 2). In some embodiments, the composite virtual image 791 is generated by the virtual visualization system 115 (FIG. 1) of the control system 112 (FIG. 1). Generating the composite virtual image 791 involves registering the image reference frame (X_{I}, Y_{I}, Z_{I}) with the surgical reference frame (X_{S}, Y_{S}, Z_{S}) and/or to the medical instrument reference frame (X_{M}, Y_{M}, Z_{M}). This registration may rotate, translate, or otherwise manipulate by rigid and/or non-rigid transforms coordinate points of the point cloud (e.g., the coordinate points 462 of the point cloud 460 of FIG. 4) captured by the positional sensor system 108 to align the coordinate points with the anatomic model 750. The registration between the image and surgical/instrument frames of reference may be achieved, for example, by using a point-based iterative closest point (ICP) technique, as described in U.S. Provisional Pat. App. Nos. 62/205,440 and No. 62/205,433. In other embodiments, the registration can be achieved using another point cloud registration technique.

Based, at least in part, on the registration, the virtual visualization system 115 can additionally or alternatively generate virtual navigational images (e.g., the virtual navigational image 792 of FIG. 7) that include a virtual depiction of patient anatomy from a viewpoint of a virtual camera on the representation 704 of the medical instrument system 104 (FIG. 3) within the anatomic model 750. In the embodiment illustrated in FIG. 7, the virtual camera of the virtual navigational image 792 is positioned at a distal portion 737 of the representation 704 such that (i) the virtual viewpoint of the virtual navigational image 792 is directed distally away from the distal portion 737 of the representation 704 and (ii) the representation 704 is not visible within the virtual navigational image 792. In other embodiments, the virtual visualization system 115 can position the virtual camera (a) at another location along the representation 704 and/or (b) in a different orientation such that the virtual navigational image 792 has a corresponding virtual viewpoint. In some embodiments, depending on the position and orientation of the virtual camera and on the positions of the elongate device 231 and the medical instrument 232 relative to one another within the patient 103, the virtual visualization system 115 can render a virtual representation (not shown) of at least a portion of the elongate device 231 and/or of the medical instrument 232 into the virtual navigational image 792.

In some embodiments, the virtual visualization system 115 can place the virtual camera within the anatomic model 750 at a position and orientation corresponding to the position and orientation of the image capture device 247 within the patient 103 (FIG. 2). As further shown in FIG. 7, the virtual navigational image 792 illustrates virtual patient anatomy, such as a carina 701 marking a branching point of two anatomic passageways 752 of the anatomic model 750, from substantially the same location at which the real navigational image 770 is captured by the image capture device 247 (FIG. 2). Thus, the virtual navigational image 792 provides a rendered estimation of patient anatomy visible to the image capture device 247 at a given location within the anatomic region 350 of FIG. 3. Because the virtual navigational image 792 is based, at least in part, on the registration of a point cloud generated by the positional sensor system 108 and image data captured by the imaging system 118, the correspondence between the virtual navigational image 792 and the real navigational image 770 provides insight regarding the accuracy of the registration and can be used to improve the registration, as described in greater detail below. Furthermore, the real navigational images (e.g., the real navigational image 770) captured by the endoscopic imaging system 109 (FIG. 2) can (a) provide information regarding the position and orientation of the medical instrument system 104 (FIG. 1) within the patient 103, (b) provide information regarding portions of an anatomic region actually visited by the medical instrument system, and/or (c) help identify patient anatomy (e.g., branching points of anatomic passageways) proximate the medical instrument system 104, any one or more of which can be used to improve the accuracy of the registration as described in greater detail below.

As further shown in FIG. 7, the virtual navigational image 792 can optionally include a navigation path overlay 799. In some embodiments, the navigation path overlay 799 is used to aid an operator 105 (FIG. 1) to navigate the medical instrument system 104 (FIG. 1) through anatomic passageways of an anatomic region to a target location within a patient 103. For example, the navigation path overlay 799 can illustrate a "best" path through an anatomic region for an operator 105 to follow to deliver the distal portions 237 and/or 238 of the medical instrument 232 and/or of the elongate device 231, respectively, to a target location within the patient 103. In some embodiments, the navigation path overlay 799 can be aligned with a centerline of or another line along (e.g., the floor of) a corresponding anatomic passageway.

### 2. Associated Methods

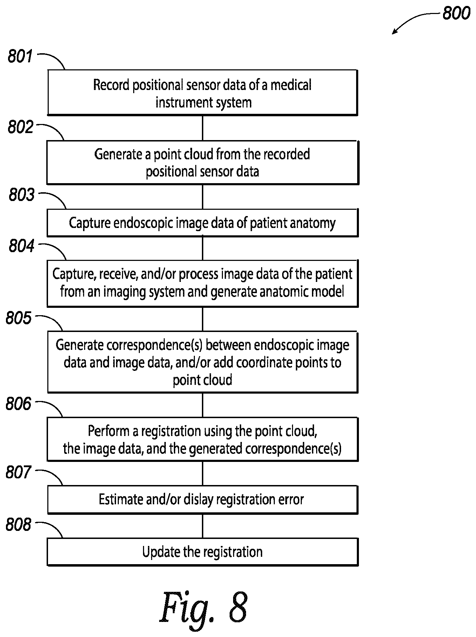

FIG. 8 is a flow diagram illustrating a method 800 for registering an image of patient anatomy to a point cloud of coordinate points using endoscopic image data in accordance with various embodiments of the present technology. The method 800 is illustrated as a set of steps or processes 801-808 and is described at least in part below with reference to FIGS. 7 and 9-13C. All or a subset of the steps of the method 800 can be executed by various components or devices of a robotic or teleoperated system, such as the system 100 illustrated in FIG. 1 or other suitable systems. For example, all or a subset of the steps of the method 800 can be executed by components or devices of (i) the manipulator assembly 102, (ii) the medical instrument system 104, (iii) the master assembly 106, (iv) the positional sensor system 108, (v) the endoscopic imaging system 109, (vi) the display system 110, (vii) the control system 112, (viii) the virtual visualization system 115, and/or (ix) the imaging system 118. Additionally, or alternatively, all or a subset of the steps of the method 800 can be executed by an operator (e.g., a physician, a user, etc.) of the system 100. Furthermore, any one or more of the steps of the method 800 can be executed in accordance with the discussion above.

At step 801, the method 800 records positional sensor data of a medical instrument system. In some embodiments, the positional sensor data is recorded using a positional sensor system (e.g., the positional sensor system 108 of FIGS. 1 and 2). The positional sensor data can be recorded during a data capture period of the positional sensor system. The data capture period can correspond to a time period during which a shape sensor and/or one or more other positional sensors of the positional sensor system are activated to collect and record positional sensor data. During the data capture period, the medical instrument system may be stationary, may be subject to commanded movement (e.g., operator-commanded advancement or bending), and/or may be passively moving (e.g., subject to no commanded movement but subject to anatomical motion from respiratory activity, cardiac activity, or other voluntary or involuntary patient motion).

As discussed in greater detail above, the positional sensor data provides position information (shape, position, orientation, pose, movement, etc.) of the medical instrument system while at least a portion of the medical instrument system is located within a patient. For example, the positional sensor data can include shape data. In these and other embodiments, the positional sensor data can include position information related to a distal end of and/or other points along an elongate device (e.g., the elongate device 231 of FIGS. 1 and 2) and/or a medical instrument (e.g., the medical instrument 232 of FIG. 2) of the medical instrument system. In some embodiments, the positional sensor data can be at least partially recorded as one or more coordinate points in two or three dimensions in a medical instrument reference frame (X_{M}, Y_{M}, Z_{M}), which is known relative to a surgical reference frame (X_{S}, Y_{S}, Z_{S}) of a surgical environment. In these and other embodiments, each coordinate point can be associated with a timestamp, which can be recorded as part of the positional sensor data.

At step 802, the method 800 generates a point cloud from the recorded positional sensor data. In some embodiments, the point cloud is generated from the union of all or a subset of the coordinate points recorded at step 801 during the data capture period of the positional sensor system. In these and other embodiments, the point cloud represents one or more shapes of the medical instrument system as the medical instrument system is stationary and/or is actively or passively moved within the patient. The point cloud may be generated in two or three dimensions in the medical instrument reference frame (X_{M}, Y_{M}, Z_{M}).

At step 803, the method 800 captures endoscopic image data of patient anatomy. In some embodiments, the endoscopic image data is captured using an endoscopic imaging system (e.g., the endoscopic imaging system 109 of FIGS. 1 and 2). The endoscopic image data can be captured during an image capture period of the endoscopic imaging system. The image capture period can correspond to a time period during which at least one image capture device of the endoscopic imaging system 109 is activated to collect and record endoscopic image data. During the image capture period, the medical instrument system may be stationary, may be subject to commanded movement (e.g., operator-commanded advancement or bending), and/or may be passively moving (e.g., subject to no commanded movement but subject to anatomical motion from respiratory activity, cardiac activity, or other voluntary or involuntary patient motion).

As discussed in greater detail above, the endoscopic image data captures one or more images (e.g., still images, video, etc.) from a viewpoint of the medical instrument system. An image capture device of the endoscopic imaging system is mounted to a distal end of the medical instrument system (e.g., to the distal portion 238 of the elongate device 231 and/or to the distal portion 237 of the medical instrument 232 of FIG. 2). Furthermore, the image capture device can be oriented such that a field of view of the image capture device is substantially parallel with an axis defined by at least a distal end portion of the medical instrument system and projected away from the medical instrument system. In these embodiments, the endoscopic image data can include one or more images of objects in front of (e.g., more distal than) the distal end of the medical instrument system. Thus, continuing with this example, when the distal end of the medical instrument system is located within an anatomic region of the patient, the endoscopic image data can include one or more real navigational images of patient anatomy in front of (e.g., distal to) the distal end of the medical instrument system. Other mounting positions and/or other orientations for the image capture device of the endoscopic imaging system are of course possible and within the scope of the present technology. In some embodiments, each real navigational image of the endoscopic image data is associated with a timestamp, which can be recorded as part of the endoscopic image data. Additionally, or alternatively, the position of the image capture device when the image capture device captures a real navigational image can be known and recorded in the medical instrument reference frame (X_{M}, Y_{M}, Z_{M}) as a part of the endoscopic image data.

At step 804, the method 800 captures, receives, and/or processes image data of the patient and generates an anatomic model. In some embodiments, the image data is captured using an imaging system (e.g., the imaging system 118 of FIGS. 1 and 2). For example, the image data can be captured using a CT imaging system. The image data can be captured, received, and/or processed during an image capture period of the imaging system. The image capture period can correspond to a time period during which the imaging system is activated. In some embodiments, the image capture period can be preoperative such that the image data is captured, received, and/or processed before the medical instrument system is advanced into the patient. In these and other embodiments, the image capture period can be intraoperative such that the image data of the patient is captured, received, and/or processed while the medical instrument system is positioned within the patient. In these embodiments, the medical instrument system may be stationary during the image capture period, may be subject to commanded movement (e.g., operator-commanded advancement or bending) during the image capture period, and/or may be passively moving (e.g., subject to no commanded movement but subject to anatomical motion from respiratory activity, cardiac activity, or other voluntary or involuntary patient motion) during the image capture period. In still other embodiments, the image capture period can be postoperative such that the image data of the patient is captured, received, and/or processed after the medical instrument system is removed from the patient. In some embodiments, the image data can be captured, received, and/or processed in real-time or near real-time.

As discussed in greater detail above, the image data of the patient includes graphical elements representing anatomical features of the patient and (in the case of intraoperative image data) graphical elements representing the medical instrument system. A model of the anatomical features of the patient is generated by segmenting and filtering the graphical elements included in the image data. During the segmentation process, pixels or voxels generated from the image data may be partitioned into segments or elements and/or be tagged to indicate that they share certain characteristics or computed properties such as color, density, intensity, and texture. In some embodiments, less than all of the image data may be segmented and filtered. The segments or elements associated with anatomical features of the patient are then converted into an anatomic model, which is generated in an image reference frame (X_{I}, Y_{I}, Z_{I}).

At step 805, the method 800 generates one or more correspondences between the endoscopic image data of patient anatomy captured at step 803 and the image data of the patient captured, received, and/or processed at step 804, and/or updates the point cloud generated at step 802 based, at least in part, on the correspondence(s). As discussed above, an image capture device of the endoscopic imaging system is mounted to a distal portion of the medical instrument system and positioned within an anatomic region of the patient. In these embodiments, the endoscopic image data captured at step 803 includes (i) images of real patient anatomy near the distal end of the medical instrument system and (ii) indications of positions of the distal portion of the medical instrument within anatomic passageways actually visited by the medical instrument system. Thus, when the method 800 determines a real navigational image of patient anatomy (e.g., a carina marking a branching point of two or more anatomic passageways) in the endoscopic image data captured at step 803 matches a portion of the image data of the patient captured, received, and/or processed at step 804, the method 800 generates a correspondence between the endoscopic image data of step 803 and the image data of step 804. Because the matched real navigational image of patient anatomy in the endoscopic image data is associated with a timestamp and a known position of the image capture device within the medical instrument frame of reference (X_{M}, Y_{M}, Z_{M}), the correspondence generated between the endoscopic image data of step 803 and the image data of step 804 provides a known correspondence between the medical instrument frame of reference (X_{M}, Y_{M}, Z_{M}) and the image reference frame (X_{I}, Y_{I}, Z_{I}) at the known position of the image capture device. The method 800 updates the point cloud generated at step 802 based, at least in part, on the generated correspondences. For example, the method 800 adds one or more coordinate points in the medical instrument frame of reference (X_{M}, Y_{M}, Z_{M}) to the point cloud of step 802 at and/or proximate the known position of the image capture device when the image capture device captured the real navigational image of the endoscopic image data that matched the image data of step 804.

At step 806, the method 800 registers the point cloud generated at step 802 and/or updated at step 805 to the anatomic model generated at step 804. In some embodiments, the registration involves aligning the medical instrument frame of reference (X_{M}, Y_{M}, Z_{M}) and/or the surgical reference frame (X_{S}, Y_{S}, Z_{S}) with the image reference frame (X_{I}, Y_{I}, Z_{I}). For example, the point cloud of steps 802 and/or 805 in the medical instrument reference frame (X_{M}, Y_{M}, Z_{M}) can be registered to the anatomic model in the image reference frame (X_{I}, Y_{I}, Z_{I}). This registration may rotate, translate, or otherwise manipulate by rigid and/or non-rigid transforms coordinate points of the point cloud (e.g., the coordinate points generated from the positional sensor data at steps 801 and 802 and/or the added coordinate points generated at step 805 from correspondences between real navigational images in the endoscopic image data of step 803 and the image data of step 804) to align the coordinate points with the anatomic model generated at 804. The transforms may be six degrees-of-freedom transforms, such that the point clouds may be translated or rotated in any or all of X, Y, Z, pitch, roll, and yaw. In some embodiments, the method 800 uses an iterative closest point (ICP) algorithm to perform the registration. In particular, the method 800 can (i) compute a point-to-point correspondence between coordinate points in the point cloud to points (e.g., on a centerline or at other locations) within the anatomic model and (ii) compute an optimal transform to minimize Euclidean distances between corresponding points. In other embodiments, the method 800 can use another technique to perform the registration.

In some embodiments, the method 800 can use the endoscopic image data captured at step 803 to improve the accuracy of and/or otherwise provide insight for the registration between the point cloud generated at step 802 and/or updated at step 805 and the anatomic model generated at step 804. For example, as discussed above with respect to step 805, the method 800 can add one or more coordinate points at known locations of the image capture device where patient anatomy in real navigational images of the endoscopic image data of step 803 matches patient anatomy captured in the image data of step 804. In some embodiments, the added coordinate points can be used in the ICP algorithm in combination with the coordinate points generated from the positional sensor data of steps 801 and/or 802 to compute the optimal transform. In these and other embodiments, the added coordinate points can be weighted differently (e.g., heavier or lighter) in the computation than the coordinate points generated from the positional sensor data of step 801. In these and still other embodiments, orientation alignment data captured by the correspondence at step 805 (e.g., information regarding how patient anatomy in a matched real navigational image of the endoscopic image data of step 803 must be transformed (e.g., translated, rotated, reflected, etc.) to align with the corresponding portion of patient anatomy in the image data of step 804) can be fed as an additional error term minimized by the registration algorithm to further inform the registration between the point cloud and the image data of step 804.

In these and other embodiments, the method 800 can use the endoscopic image data captured at step 803 to temporally or locally improve the accuracy of and/or otherwise provide insight for the registration performed at step 806. For example, the method 800 can use coordinate points added at step 805 and/or orientation alignment data captured by the correspondence at step 805 to improve the accuracy of and/or otherwise provide insight for only a portion of the registration performed at step 806. Continuing with this example, the portion of the registration performed at step 806 can correspond to coordinate points from steps 802 and/or 805 and/or a subset of points of the anatomic model generated at step 804 within threshold distances of coordinate points added at step 805 and/or within threshold distances of correspondences generated at step 805.

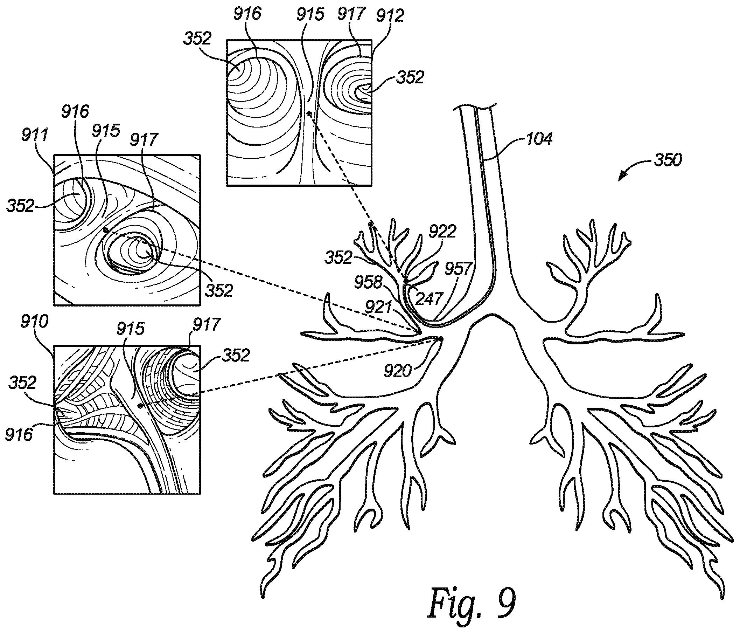

Alternatively, the method 800 can perform a registration (e.g., a sparse point registration) between only (a) coordinate points stemming from the endoscopic image data of step 803 and (b) the anatomic model generated at step 804. FIG. 9, for example, is a schematic representation of (i) the anatomic region 350 (e.g., lungs) of a patient illustrated in FIG. 3 and (ii) real navigational images 910-912 of patient anatomy within the anatomic region 350 captured by an image capture device 247 of the medical instrument system 104 as endoscopic image data at step 803. As shown, the real navigational images 910-912 are images of branching points 920-922 of anatomic passageways 352 captured as the medical instrument system 104 is navigated throughout the anatomic region 350. The branching points 920-922 of the anatomic region 350 are anatomic features that are readily recognizable in real navigational images of the endoscopic image data of step 803 in that they each include a bright ridge point 915 of a carina and two or more openings (e.g., openings 916 and 917) of anatomic passageways 352 in a center area of each of the real navigational images 910-912. Thus, in some embodiments, the method 800 can (e.g., automatically) identify the branching points 920-922 in the real navigational images 910-912, respectively, and/or other branching points in other real navigational images (not shown) of the endoscopic image data of step 803 and can record one or more coordinate points in a point cloud at locations corresponding to the location of the image capture device 247 when the image capture device 247 captured each of the respective real navigational images 910-912. The point cloud can be the point cloud generated at step 802 and/or updated at step 805, and/or another point cloud. Continuing with the above example with reference to FIG. 9, the method 800 can perform a sparse point registration between (i) the anatomic model generated at step 804 and (ii) only coordinate points stemming from the real navigational images 910-912 and/or other real navigational images (not shown) in the endoscopic image data of step 803 in which the method 800 has identified a branching point of anatomic passageways 352.

In these and other embodiments, the method 800 can use the real navigational images of the endoscopic image data of step 803 to provide insight as to the pathway taken by the medical instrument system as it is navigated throughout an anatomic region. For example, after the method 800 identifies a branching point in a real navigational image of the endoscopic image data of step 803, the method 800 can use the real navigational image and/or one or more real navigational images previously and/or subsequently captured in the endoscopic image data to determine which of the anatomic passageways of the branching point the medical instrument system took as it navigated throughout the anatomic region.

As a more specific example with continuing reference to FIG. 9, after the method 800 identifies the branching point 920 in the real navigational image 910, the method 800 can use the real navigational image 910 and one or more real navigational images previously and/or subsequently captured in the endoscopic image data of step 803 to determine whether the medical instrument system 104 traversed through the opening 916 or through the opening 917. In this case, the method 800 can determine that the medical instrument system 104 traversed through the opening 917 of the right anatomic passageway 352. In other words, the endoscopic image data of step 803 can be used to estimate a specific path taken by the medical instrument system 104 throughout the anatomic region 350. In turn, the method 800 can use this information to instruct the ICP algorithm to register data points of the point cloud (e.g., the point cloud of sparse points, the point cloud of step 802, and/or the point cloud of step 805) to a specific region (e.g., to a region corresponding to the right anatomic passageway 352 in the real navigational image 910) of the anatomic model generated at step 804. Thus, this information can be used to improve registration accuracy in comparison to a naive ICP algorithm that would otherwise register data points of the point cloud to the nearest region (e.g., to a region corresponding to the left anatomic passageway 352 of the real navigational image 910) of the anatomic model regardless of whether another less-proximate region (e.g., the region corresponding to the right anatomic passageway 352 of the real navigational image 910) of the anatomic model actually corresponds to the data points of the point cloud. This is expected to particularly help improve registration when the two regions are closely spaced from one another in the anatomic model.

When the medical instrument reference frame (X_{M}, Y_{M}, Z_{M}) is registered to the image reference frame (X_{I}, Y_{I}, Z_{I}), images displayed to an operator on the display system may allow the operator to more accurately steer the medical instrument system through patient anatomy, observe the patient anatomy from the perspective of a distal end of the medical instrument system, and/or improve efficiency and efficacy of targeted medical procedures. For example, the method 800 in some embodiments can display a composite virtual image (e.g., the composite virtual image 791 of FIG. 7) that includes the anatomic model generated at step 804 with a representation of the medical instrument system having a position, a shape, an orientation, a pose, and/or a movement (e.g., speed, velocity, etc.) within the anatomic model that corresponds to a position, a shape, an orientation, a pose, and/or a movement of the medical instrument system within the patient. For example, the representation of the medical instrument system can be superimposed on the anatomic model.

In these and other embodiments, based, at least in part, on the performed registration, the method 800 can calculate a real-time and/or near real-time virtual navigational image (e.g., the virtual navigational image 792 of FIG. 7) at a location within the anatomic model that corresponds to a location of an image capture device of the medical instrument system within the patient. For example, the method 800 can compute a virtual navigational image corresponding to a real navigational image in the endoscopic image data of step 803. In some embodiments, the method 800 can select for which of the real navigational images of the endoscopic image data to compute a corresponding virtual navigational image. As discussed above, branching points of anatomic passageways are readily recognizable patient anatomy in the real navigational images. Thus, the method 800 in some embodiments can select only those real navigational images in which the method 800 identifies a branching point or other recognizable patient anatomy for which to compute corresponding virtual navigational images. In other embodiments, the method 800 can use other selection criteria. The method 800 can display the computed virtual navigational images and/or real navigational images (e.g., the real navigational image 770 of FIG. 7) of the endoscopic image data of step 803 on a display of the display system.

At step 807, the method 800 estimates and/or displays a registration error for the registration performed at step 806. For example, the method 800 can compute a disagreement between (i) a known position of the image capture device associated with a real navigational image of the endoscopic image data of step 803 that matches the image data of step 804 and (ii) the estimated position of the image capture device within the registration generated at step 806. After computing the disagreement, the method 800 can display the estimated registration error on a display of the display system.

For the sake of clarity and understanding of the above concept, consider the following additional example with reference to both FIGS. 7 and 9. After performing the registration at step 806, the method 800 can display a composite virtual image 791 (FIG. 7) illustrating the anatomic model 750 (FIG. 7) generated at step 804 with a representation 704 (FIG. 7) of the medical instrument system 104 (FIG. 9). The method 800 can then compute an estimated registration error for (i) a portion 757 (FIG. 7) of an anatomic passageway 752 (FIG. 7) of the composite virtual image 791 that corresponds to a portion 957 (FIG. 9) of an anatomic passageway 352 (FIG. 9) of the anatomic region 350 (FIG. 9) and (ii) a portion 758 (FIG. 7) of the anatomic passageway 752 of the composite virtual image 791 that corresponds to a portion 958 (FIG. 9) of the anatomic passageway 352 of the anatomic region 350. In this example, the method 800 can display the estimated registration errors by varying colors, patterns, and/or other visual indicators (e.g., numerical displays) of the portions 757 and 758 within the composite virtual image 791 in accordance with the magnitudes of the respective registration errors. For example, the method 800 can color the portion 757 of the anatomic model 750 in the composite virtual image 791 green to indicate that the magnitude of the estimated registration error at that portion 757 of the composite virtual image 791 is relatively small (e.g., to indicate that the registration of the point cloud to the image data of step 804 at that location aligns well with the correspondence between the endoscopic image data of step 803 and a portion of the image data of step 804 at that location). In contrast, the method 800 can color the portion 758 of the anatomic model 750 in the composite virtual image 791 a different (e.g., a fainter, less intense, less bright) shade of green or a different color (e.g., yellow, orange, red, etc.), pattern, and/or visual indicator altogether to indicate that the magnitude of the estimated registration error at the portion 758 is relatively large (e.g., to indicate that the registration of the point cloud to the image data of step 804 does not align as well with the correspondence between the endoscopic image data of step 803 and a portion of the image data of step 804 at that location). In this manner, the method 800 can display a gradient of colors, patterns, and/or other visual indicators (e.g., numeric displays) within the composite virtual image 791 to indicate the estimated registration errors across the anatomic model 750. This can be useful, for example, in determining a best path through patient anatomy to a target location and/or for determining whether current patient anatomy aligns with preoperative imaging of the patient at a portion of interest in the anatomic model 750.

In these and other embodiments, the method 800 can estimate and/or display a registration error in real-time or near real-time. For example, the method 800 can estimate a registration error in real-time or near real-time for a current location of an image capture device of the medical instrument system within the patient. In this example, the method 800 can compute a disagreement at or proximate the current location of the image capture device between (i) a position of the image capture device associated with a real navigational image of the endoscopic image data of step 803 that matches the image data of step 804 and (ii) the estimated position of the image capture device within the registration performed at step 806.

After computing the disagreement, the method 800 can display the estimated registration error in real-time or near real-time on a display of the display system. Referring again to FIG. 7 for the sake of example, the method 800 can vary, in real-time, a color, pattern, and/or other visual indicator of the portion 757 of the anatomic model 750 within the composite virtual image 791 at or proximate the current location of an image capture device (e.g., at or proximate the current location of the distal portion 737 of the representation 704 of the medical instrument system). Thus, over a time period during which the patient is breathing, a sequence of colors, shades, patterns, and/or other visual indicators can be used to display the portion 757 to indicate the change in magnitude of an estimated registration error over that time period. In other words, the method 800 can provide a temporal indication of when the registration of the point cloud to the image data of step 804 at a given location aligns well with a correspondence between the endoscopic image data of step 803 and a portion of the image data of step 804 at that given location. This information can be useful, for example, in providing a temporal indication of where to gate a patient's respiratory phase and take a biopsy of target tissue with a breath hold.

In these and other embodiments, the method 800 can vary a color, pattern, and/or other visual indicators of other information on the display to indicate an estimated registration error in real-time, near real-time, or otherwise. For example, the method 800 can vary a color, pattern, and/or other visual indicator used to display virtual patient anatomy in a virtual navigational image (e.g., the virtual navigational image 792 of FIG. 7) and/or used to display a navigation path overlay (e.g., the navigation path overlay 799 of FIG. 7) within the virtual navigational image.

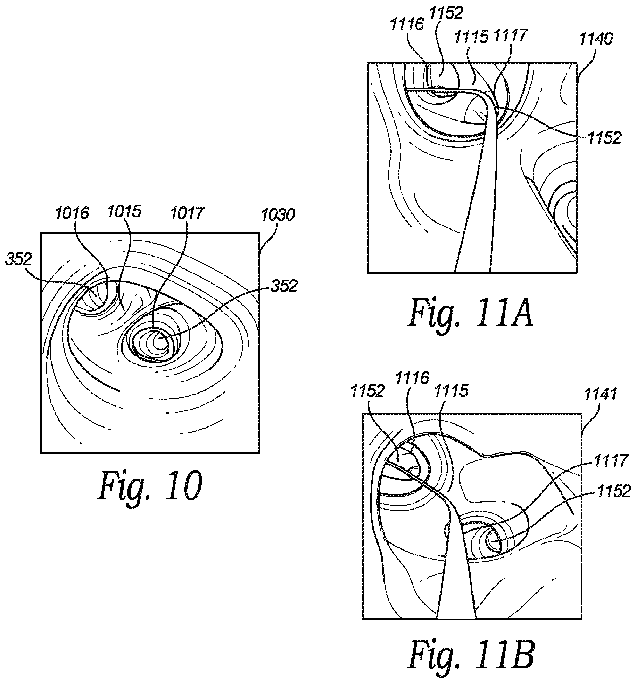

At step 808, the method 800 updates the registration performed at step 806. In some embodiments, the method 800 can update the registration by returning to step 801 and reperforming (e.g., iteratively performing) all or a subset of the steps 801-807. In these and other embodiments, the method 800 can update the registration performed at step 806 using the endoscopic image data captured at step 803. For example, the method 800 can use one or more real navigational images of the endoscopic image data of step 803 to align computed virtual navigational images to corresponding real navigational images of the endoscopic image data. For the sake of clarity and understanding, consider the following example with reference to FIGS. 10-11B. FIG. 10 is a real navigational image 1030 of real patient anatomy captured in the endoscopic image data of step 803. The real patient anatomy in the real navigational image 1030 includes a carina 1015 marking a branching point of two anatomic passageways 352. The openings 1016 and 1017 of the anatomic passageways 352 are visible in the real navigational image 1030.

In some embodiments, the method 800 can compute a virtual navigational image based, at least in part, on the registration performed at step 806 at a location corresponding to the location of the image capture device when the image capture device captured the real navigational image 1030. FIG. 11A, for example, is a virtual navigational image 1140 of virtual patient anatomy computed by the method 800 based, at least in part, on the registration performed at step 806 at a location corresponding to the location of the image capture device in the real navigational image 1030 of FIG. 10. As shown, the virtual patient anatomy in the virtual navigational image 1140 includes a carina 1115 marking a branching point of two virtual anatomic passageways 1152. The openings 1116 and 1117 of the anatomic passageways 1152 are visible in the virtual navigational image 1140. The branching point of the virtual patient anatomy in the virtual navigational image 1140 corresponds to the branching point of the real patient anatomy in the real navigational image 1030 of FIG. 10.

Referring to FIGS. 10 and 11A together, the method 800 can determine that the virtual patient anatomy in the computed virtual navigational image 1140 (FIG. 11A) does not align with the real patient anatomy in the real navigational image 1030 (FIG. 10). In other words, the method 800 can determine that the registration performed at step 806 does not align with the endoscopic image data of step 803 at the location of the image capture device associated with the real navigational image 1030. In these embodiments, the method 800 can compute a transform to align the virtual navigational image 1140 to the real navigational image 1030. For example, the method 800 can determine that the method 800 must translate the position of the virtual image capture device associated with the virtual navigational image 1140 forward and rotate it slightly counter-clockwise to align the virtual navigational image 1140 with the real navigational image 1030. FIG. 11B is a virtual navigational image 1141 of the virtual patient anatomy of FIG. 11A after performing this transformation. In some embodiments, the computed transformation can be used as a delta registration matrix to update the registration (e.g., the ICP registration) performed at step 806. This can involve changing a position of a coordinate point generated from the positional sensor data of step 801 and recorded in the point cloud of step 802 and/or step 805 from a location corresponding to the position of the virtual image capture device associated with the virtual navigational image 1140 (FIG. 11A) to a location corresponding to the position of the virtual image capture device associated with the virtual navigational image 1141 (FIG. 11B).

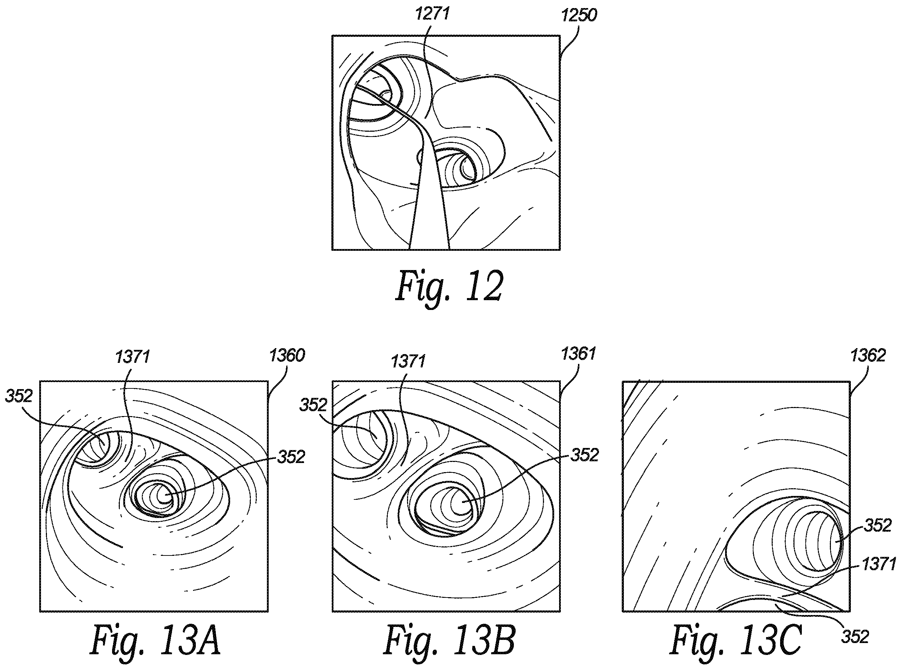

In these and still other embodiments, the method 800 (at step 808) can update the registration performed at step 806 by correcting the registration for drift away from the endoscopic image data of step 803. For the sake of clarity and understanding of this concept, consider the following example with reference to FIGS. 12-13C. FIG. 12, for example, is a virtual navigational image 1250 of virtual patient anatomy 1271, and FIGS. 13A-13C illustrate a sequence of consecutive real navigational images 1360-1362 of real patient anatomy 1371 captured in the endoscopic image data of step 803. In this example, the method 800 (i) recognizes the real patient anatomy 1371 in the real navigational image 1360 (FIG. 13A) includes a branching point of two anatomic passageways 352 and (ii) computes the virtual navigational image 1250 (FIG. 12) based, at least in part, on the registration performed at step 806 at a location within a generated anatomic model (not shown) corresponding to the location of the image capture device in the real navigational image 1360 (FIG. 13A).

The virtual patient anatomy 1271 in the virtual navigational image 1250 of FIG. 12 does not fully align with the real patient anatomy 1371 in the real navigational image 1360 of FIG. 13A. As discussed above, each virtual navigational image (including the virtual navigational image 1250 of FIG. 12) and each real navigational image (including the real navigational images 1360-1362 of FIGS. 13A-13C) is associated with a timestamp indicating the point in time a corresponding portion of data (e.g., positional sensor data of step 801 and/or endoscopic image data of step 803) was captured. Therefore, the method 800 can search the real navigational images (including the real navigational images 1360-1362) of the endoscopic image data of step 802 captured within a period of time surrounding (e.g., having a timestamp occurring before, during, and/or after) the timestamp associated with the virtual navigational image 1250 for a real navigational image that best matches the virtual navigational image 1250. In this example, the real navigational image 1361 of FIG. 13B best matches the virtual navigational image 1250 of FIG. 12. The method 800 can then compute a difference between the timestamp of the best matching real navigational image 1361 and the timestamp of the virtual navigational image 1250 and use the difference as a delta registration matrix to update the registration (e.g., the ICP registration) performed at step 806. For example, the method 800 can change a position of one or more coordinate points in the point cloud of step 802 and/or in the point cloud of step 805 that correspond to the virtual navigational image 1250 of FIG. 12 to the position of the image capture device associated with the best matching real navigational image 1361 of FIG. 13B.

Although the above concept is illustrated and discussed above in the context of matching a branching point of two anatomic passageways in a virtual navigational image with corresponding patient anatomy in real navigational images, the above concept is particularly useful in locations where a branching point is not visible in the virtual and real navigational images. For example, a diameter of an anatomic passageway typically decreases as the medical instrument system navigates further along it. Thus, the above concept can be used to determine a real navigational image that illustrates an anatomic passageway with a diameter that best matches a diameter of the anatomic passageway in a virtual navigational image. The best match, therefore, can provide information regarding how far into an anatomic passageway the medical instrument system has been inserted at a given point in time.

The method 800, at step 808, temporally or locally updates the registration performed at step 806. For example, the method 800 can update the registration performed at step 806 for a specific respiratory or cardiac phase. Continuing with this example, the method 800 can update the registration performed at step 806 differently for a different respiratory or cardiac phase. As another example, the method 800 can, at step 808, update only a portion of the registration performed at step 806. Continuing with this example, the portion of the registration updated can correspond to coordinate points from steps 802 and/or 805 and/or a subset of points of the anatomic model generated at step 804 within threshold distances of coordinate points and/or anatomic model points corresponding to one or more real and/or virtual navigational images.

Some of the computations (e.g., matching between real and virtual navigational images) performed in the steps 801-808 above can be particularly resource intensive. Thus, as an extension of any one or more of the steps 801-808 discussed above, the method 800 can use the endoscopic image data captured at step 802 and/or other information available to the method 800 to determine when to perform certain computations of the method 800. In some embodiments, the method 800 can use input/output values of the medical instrument system to identify when to perform registration computations. For example, the method 800 can use distance traversed by a distal end of the medical device as an indicator of when to perform computations. As a more specific example, the method 800 can anticipate that a patient's main carina lays approximately a first distance away from a distal end of the medical instrument system at a point of initial insertion into the patient. Thus, the method 800 can monitor and identify when the distal end of the medical instrument system has traversed the first distance from the point of initial insertion to determine when to attempt to capture the main carina in endoscopic image data and/or when to attempt to generate a correspondence between real navigational images of the endoscopic image data and (e.g., preoperative) image data of the patient's main carina. Additionally, or alternatively, the method 800 can use motion of the positional sensor system and/or the registration performed at step 806 to estimate when an image capture device of the endoscopic imaging system is likely near a carina and can use this estimation to determine when to attempt to generate a correspondence between real navigational images of the endoscopic image data captured at step 802 and the (e.g., preoperative and/or intraoperative) image data of the patient captured, received, and/or processed at step 804.

In these and other embodiments, the method 800 can use the occurrence of other events to determine when to perform computations. For example, the method 800 can perform specific computations each time the distal end or another portion of the medical instrument system traverses a threshold distance (e.g., each time the position of the distal end changes by a threshold amount). As another example, the method 800 can perform specific computations after the orientation of the distal end of the medical instrument system has changed by a threshold amount. As yet another example, the method 800 can capture positional sensor data and/or endoscopic image data periodically (e.g., in accordance with set intervals and/or events) and can wait to perform resource intensive computations until the method 800 determines the medical instrument system is subject to commanded movement (e.g., by an operator) and/or until another event occurs.

Although the steps of the method 800 are discussed and illustrated in a particular order, the method 800 illustrated in FIG. 8 is not so limited. In other embodiments, the method 800 can be performed in a different order. For example, the steps 804 can be performed any one of the steps 801-803. In these and other embodiments, any of the steps of the method 800 can be performed before, during, and/or after any of the other steps of the method 800. Moreover, a person of ordinary skill in the relevant art will recognize that the illustrated method 800 can be altered and still remain within these and other embodiments of the present technology. For example, one or more steps of the method 800 illustrated in FIG. 8 can be omitted and/or repeated in some embodiments.

### B. Conclusion

The systems and methods described herein can be provided in the form of tangible and non-transitory machine-readable medium or media (such as a hard disk drive, hardware memory, etc.) having instructions recorded thereon for execution by a processor or computer. The set of instructions can include various commands that instruct the computer or processor to perform specific operations such as the methods and processes of the various embodiments described here. The set of instructions can be in the form of a software program or application. The computer storage media can include volatile and non-volatile media, and removable and non-removable media, for storage of information such as computer-readable instructions, data structures, program modules or other data. The computer storage media can include, but are not limited to, RAM, ROM, EPROM, EEPROM, flash memory or other solid-state memory technology, CD-ROM, DVD, or other optical storage, magnetic disk storage, or any other hardware medium which can be used to store desired information and that can be accessed by components of the system. Components of the system can communicate with each other via wired or wireless communication. The components can be separate from each other, or various combinations of components can be integrated together into a monitor or processor or contained within a workstation with standard computer hardware (for example, processors, circuitry, logic circuits, memory, and the like). The system can include processing devices such as microprocessors, microcontrollers, integrated circuits, control units, storage media, and other hardware.

Although many of the embodiments are described above in the context of navigating and performing medical procedures within lungs of a patient, other applications and other embodiments in addition to those described herein are within the scope of the present technology. For example, unless otherwise specified or made clear from context, the devices, systems, methods, and computer program products of the present technology can be used for various image-guided medical procedures, such as medical procedures performed on, in, or adjacent hollow patient anatomy, and, more specifically, in procedures for surveying, biopsying, ablating, or otherwise treating tissue within and/or proximal the hollow patient anatomy. Thus, for example, the systems, devices, methods, and computer program products of the present disclosure can be used in one or more medical procedures associated with other patient anatomy, such as the bladder, urinary tract, GI system, and/or heart of a patient.

As used herein, the term "operator" shall be understood to include any type of personnel who may be performing or assisting a medical procedure and, thus, is inclusive of a physician, a surgeon, a doctor, a nurse, a medical technician, other personnel or user of the technology disclosed herein, and any combination thereof. Additionally, or alternatively, the term "patient" should be considered to include human and/or non-human (e.g., animal) patients upon which a medical procedure is being performed.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms can also include the plural or singular term, respectively. Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. As used herein, the phrase "and/or" as in "A and/or B" refers to A alone, B alone, and both A and B. Where the context permits, singular or plural terms can also include the plural or singular term, respectively. Additionally, the terms "comprising," "including," "having" and "with" are used throughout to mean including at least the recited feature(s) such that any greater number of the same feature and/or additional types of other features are not precluded.

Furthermore, as used herein, the term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result.

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments can perform steps in a different order. As another example, various components of the technology can be further divided into subcomponents, and/or various components and/or functions of the technology can be combined and/or integrated. Furthermore, although advantages associated with certain embodiments of the technology have been described in the context of those embodiments, other embodiments can also exhibit such advantages, and not all embodiments need necessarily exhibit such advantages to fall within the scope of the technology.

It should also be noted that other embodiments in addition to those disclosed herein are within the scope of the present technology. For example, embodiments of the present technology can have different configurations, components, and/or procedures in addition to those shown or described herein. Moreover, a person of ordinary skill in the art will understand that these and other embodiments can be without several of the configurations, components, and/or procedures shown or described herein without deviating from the present technology. Accordingly, the disclosure and associated technology can encompass other embodiments not expressly shown or described herein.

## Claims

1. A medical system for use in an image-guided medical procedure, the system comprising:
a positional sensor configured to generate, in a medical instrument frame of reference, positional sensor data associated with one or more positions of a medical device within an anatomic region of a patient;
the medical device;
an image capture device (247) at a distal portion of the medical device, the image capture device configured to capture, from one or more known positions in the medical instrument frame of reference, first image data of patient anatomy within the anatomic region while the medical device is positioned within the anatomic region;
a processor (114) communicatively coupled to the positional sensor and the image capture device (247); and
a memory (116) storing instructions that, when executed by the processor (114), cause the system to perform operations comprising-
generating a point cloud of coordinate points based, at least in part, on the positional sensor data,
receiving second image data of the anatomic region, wherein the second image data is generated based, at least in part, on imaging of the anatomic region,
generating one or more correspondences by matching patient anatomy in one or more images of the first image data with patient anatomy of the anatomic region in the second image data,
generating a registration between at least a portion of the point cloud and at least a portion of the second image data, and
updating the registration based, at least in part, on the first image data,
**characterized in that** the instructions further cause the system to perform operations comprising:
adding, before generating the registration, one or more coordinate points to the point cloud at one or more locations corresponding to the one or more known positions of the image capture device when, based on the one or more correspondences, the patient anatomy in the first image data and the patient anatomy in the second image data matched.

2. The system of claim 1 wherein the patient anatomy in the one or more images of the first image data and the patient anatomy of the anatomic region in the portion of the second image data are one or more branching points of anatomic passageways in the anatomic region.

3. The system of claim 1 wherein generating the registration includes weighting the one or more added coordinate points differently than other coordinate points of the point cloud generated from the positional sensor data.

4. The system of claim 1 wherein the portion of the point cloud includes only the one or more added coordinate points.

5. The system of claim 1 wherein the operations further comprise determining a transformation to align an image of the one or more images of the first image data with corresponding patient anatomy of the anatomic region in the portion of the second image data, and wherein updating the registration includes updating the registration based, at least in part, on the transformation.

6. The system of claim 1 wherein the operations further comprise determining, based, at least in part, on the first image data, at least a portion of a pathway taken by the medical device throughout the anatomic region, and wherein generating the registration includes generating the registration between at least the portion of the point cloud and a section of the anatomic region corresponding to the portion of the pathway.

7. The system of claim 1 wherein the operations further comprise estimating a registration error between a correspondence of the one or more correspondences and the generated registration.

8. The system of claim 7 wherein the operations further comprise coloring a display of the generated registration based, at least in part, on a magnitude of the estimated registration error.

9. The system of any of claims 1-4 and 6-8, wherein the operations further comprise:
computing, based, at least in part, on the generated registration, a virtual image of patient anatomy of the anatomic region from a perspective of the image capture device at a current location of the image capture device within the anatomic region; and
determining a transformation to align the virtual image with an image of the first image data corresponding to the current location of the image capture device.

10. The system of claim 9 wherein updating the registration includes updating the registration based, at least in part, on the determined transformation.

11. The system of claim 9 wherein the determining the transformation includes:
determining the transformation for only a portion of the generated registration within a threshold distance from the current location of the image capture device (247); or
determining the transformation for a specific respiratory and/or cardiac phase of the patient.

12. The system of any of claims 1-4 and 6-8, wherein the operations further comprise:
computing, based, at least in part, on the generated registration, a virtual image of patient anatomy of the anatomic region from a perspective of the image capture device (247) at a current or previous location of the image capture device (247) within the anatomic region, wherein the virtual image is associated with a first timestamp; and
determining an image of the first image data that best matches the virtual image, wherein the image of the first image data is included in a group of two or more images of the first image data, and wherein each image of the two or more images is associated with a timestamp occurring within a specified time period before, during, and/or after the first timestamp.

13. The system of claim 12 wherein the operations further comprise determining a difference between (i) a timestamp associated with the image of the first image data that best matches the virtual image and (ii) the first timestamp, and wherein updating the registration includes updating the registration based, at least in part, on the determined difference.

## Patentansprüche

1. Medizinisches System zur Verwendung in einem bildgesteuerten medizinischen Verfahren, wobei das System umfasst:
einen Positionssensor, der so konfiguriert ist, dass er in einem Referenzrahmen eines medizinischen Instruments Positionssensordaten erzeugt, die mit einer oder mehreren Positionen einer medizinischen Vorrichtung innerhalb eines anatomischen Bereichs eines Patienten verbunden sind;
die medizinische Vorrichtung;
eine Bilderfassungsvorrichtung (247) an einem distalen Abschnitt der medizinischen Vorrichtung, wobei die Bilderfassungsvorrichtung so konfiguriert ist, dass sie von einer oder mehreren bekannten Positionen im Referenzrahmen des medizinischen Instruments erste Bilddaten der Anatomie des Patienten innerhalb des anatomischen Bereichs erfasst, während die medizinische Vorrichtung innerhalb des anatomischen Bereichs positioniert ist;
einen Prozessor (114), der kommunikativ mit dem Positionssensor und der Bilderfassungsvorrichtung (247) verbunden ist; und
einen Speicher (116), der Anweisungen speichert, die, wenn sie von dem Prozessor (114) ausgeführt werden, das System veranlassen, Operationen durchzuführen, die umfassen:
Erzeugen einer Punktwolke von Koordinatenpunkten, die zumindest teilweise auf den Positionssensordaten basiert,
Empfangen von zweiten Bilddaten des anatomischen Bereichs, wobei die zweiten Bilddaten zumindest teilweise basierend auf der Bildgebung des anatomischen Bereichs erzeugt werden,
Erzeugen einer oder mehrerer Korrespondenzen durch Abgleichen der Patientenanatomie in einem oder mehreren Bildern der ersten Bilddaten mit der Patientenanatomie des anatomischen Bereichs in den zweiten Bilddaten,
Erzeugen einer Registrierung zwischen mindestens einem Teil der Punktwolke und mindestens einem Teil der zweiten Bilddaten, und
Aktualisieren der Registrierung, zumindest teilweise, basierend auf den ersten Bilddaten,
**dadurch gekennzeichnet, dass** die Anweisungen ferner das System veranlassen, Operationen durchzuführen, die umfassen:
Hinzufügen, bevor die Registrierung erzeugt wird, eines oder mehrerer Koordinatenpunkte zu der Punktwolke an einem oder mehreren Orten, die den einen oder mehreren bekannten Positionen der Bilderfassungsvorrichtung entsprechen, wenn, basierend auf der einen oder mehreren Korrespondenzen, die Patientenanatomie in den ersten Bilddaten und die Patientenanatomie in den zweiten Bilddaten übereinstimmen.

2. System nach Anspruch 1, wobei die Patientenanatomie in dem einen oder den mehreren Bildern der ersten Bilddaten und die Patientenanatomie des anatomischen Bereichs in dem Teil der zweiten Bilddaten ein oder mehrere Verzweigungspunkte von anatomischen Durchgängen in dem anatomischen Bereich sind.

3. System nach Anspruch 1, wobei das Erzeugen der Registrierung das Gewichten des einen oder der mehreren hinzugefügten Koordinatenpunkte anders als andere Koordinatenpunkte der aus den Positionssensordaten erzeugten Punktwolke umfasst.

4. System nach Anspruch 1, wobei der Teil der Punktwolke nur den einen oder die mehreren hinzugefügten Koordinatenpunkte enthält.

5. System nach Anspruch 1, wobei die Operationen ferner das Bestimmen einer Transformation zum Ausrichten eines Bildes des einen oder der mehreren Bilder der ersten Bilddaten mit der entsprechenden Patientenanatomie des anatomischen Bereichs in dem Teil der zweiten Bilddaten umfassen, und wobei das Aktualisieren der Registrierung das Aktualisieren der Registrierung zumindest teilweise basierend auf der Transformation umfasst.

6. System nach Anspruch 1, wobei die Operationen ferner das Bestimmen, basierend, zumindest teilweise, auf den ersten Bilddaten, zumindest eines Abschnitts eines von der medizinischen Vorrichtung durch den anatomischen Bereich genommenen Pfades umfassen, und wobei das Erzeugen der Registrierung das Erzeugen der Registrierung zwischen zumindest dem Teil der Punktwolke und einem Abschnitt des anatomischen Bereichs, der dem Teil des Weges entspricht, umfasst.

7. System nach Anspruch 1, wobei die Operationen ferner das Schätzen eines Registrierungsfehlers zwischen einer Entsprechung der einen oder mehreren Entsprechungen und der erzeugten Registrierung umfassen.

8. System nach Anspruch 7, wobei die Operationen ferner das Einfärben einer Anzeige der erzeugten Registrierung umfassen, die zumindest teilweise auf einer Größe des geschätzten Registrierungsfehlers basiert.

9. System nach einem der Ansprüche 1-4 und 6-8, wobei die Operationen ferner umfassen:
Berechnen, basierend, zumindest teilweise, auf der erzeugten Registrierung, eines virtuellen Bildes der Patientenanatomie des anatomischen Bereichs aus einer Perspektive der Bilderfassungsvorrichtung an einem aktuellen Ort der Bilderfassungsvorrichtung innerhalb des anatomischen Bereichs; und
Bestimmen einer Transformation, um das virtuelle Bild mit einem Bild der ersten Bilddaten auszurichten, das dem aktuellen Standort der Bilderfassungsvorrichtung entspricht.

10. System nach Anspruch 9, wobei das Aktualisieren der Registrierung das Aktualisieren der Registrierung zumindest teilweise basierend auf der bestimmten Transformation umfasst.

11. System nach Anspruch 9, wobei das Bestimmen der Transformation umfasst:
Bestimmen der Transformation nur für einen Teil der erzeugten Registrierung innerhalb eines Schwellenabstandes von der aktuellen Position der Bilderfassungsvorrichtung (247); oder
Bestimmen der Transformation für eine bestimmte Atmungs- und/oder Herzphase des Patienten.

12. System nach einem der Ansprüche 1-4 und 6-8, wobei die Operationen ferner umfassen:
Berechnen, basierend, zumindest teilweise, auf der erzeugten Registrierung, eines virtuellen Bildes der Patientenanatomie des anatomischen Bereichs aus einer Perspektive der Bilderfassungsvorrichtung (247) an einem aktuellen oder vorherigen Ort der Bilderfassungsvorrichtung (247) innerhalb des anatomischen Bereichs, wobei das virtuelle Bild mit einem ersten Zeitstempel verbunden ist; und
Bestimmen eines Bildes der ersten Bilddaten, das am besten mit dem virtuellen Bild übereinstimmt, wobei das Bild der ersten Bilddaten in einer Gruppe von zwei oder mehr Bildern der ersten Bilddaten enthalten ist, und wobei jedes Bild der zwei oder mehr Bilder mit einem Zeitstempel verknüpft ist, der innerhalb einer bestimmten Zeitspanne vor, während und/oder nach dem ersten Zeitstempel auftritt.

13. System nach Anspruch 12, wobei die Operationen ferner das Bestimmen einer Differenz zwischen (i) einem Zeitstempel, der dem Bild der ersten Bilddaten zugeordnet ist, das am besten mit dem virtuellen Bild übereinstimmt, und (ii) dem ersten Zeitstempel umfassen, und wobei das Aktualisieren der Registrierung das Aktualisieren der Registrierung zumindest teilweise basierend auf der bestimmten Differenz umfasst.

## Revendications

1. Système médical pour une utilisation dans une procédure médicale guidée par l'image, le système comprenant :
un capteur de position configuré pour générer, dans un cadre de référence d'instrument médical, des données de capteur de position associées à une ou plusieurs positions d'un dispositif médical au sein d'une région anatomique d'un patient ;
le dispositif médical ;
un dispositif de capture d'image (247) au niveau d'une portion distale du dispositif médical, le dispositif de capture d'image étant configuré pour capturer, à partir d'une ou de plusieurs positions connues dans le cadre de référence d'instrument médical, des premières données d'image d'anatomie de patient au sein de la région anatomique tandis que le dispositif médical est positionné au sein de la région anatomique ;
un processeur (114) couplé en communication au capteur de position et au dispositif de capture d'image (247) ; et
une mémoire (116) stockant des instructions qui, lorsqu'elles sont exécutées par le processeur (114), amènent le système à réaliser des opérations comprenant
la génération d'un nuage de points de points de coordonnées sur la base, au moins en partie, des données de capteur de position,
la réception de deuxièmes données d'image de la région anatomique, dans lequel les deuxièmes données d'image sont générées, sur la base, au moins en partie, de l'imagerie de la région anatomique,
la génération d'une ou de plusieurs correspondances par la mise en concordance de l'anatomie de patient dans une ou plusieurs images des premières données d'image avec l'anatomie de patient de la région anatomique dans les deuxièmes données d'image,
la génération d'un calage entre au moins une portion du nuage de points et au moins une portion des deuxièmes données d'image, et
la mise à jour du calage sur la base, au moins en partie, des premières données d'image,
**caractérisé en ce que** les instructions amènent en outre le système à réaliser des opérations comprenant :
l'ajout, avant la génération du calage, d'un ou de plusieurs points de coordonnées au nuage de points à un ou plusieurs emplacements correspondant aux une ou plusieurs positions connues du dispositif de capture d'image lorsque, sur la base des une ou plusieurs correspondances, l'anatomie de patient dans les premières données d'image et l'anatomie de patient dans les deuxièmes données d'image concordent.

2. Système selon la revendication 1 dans lequel l'anatomie de patient dans les une ou plusieurs images des premières données d'image et l'anatomie de patient dans la région anatomique dans la portion des deuxièmes données d'image sont un ou plusieurs points de ramification de voies de passage anatomiques dans la région anatomique.

3. Système selon la revendication 1 dans lequel la génération du calage inclut la pondération des un ou plusieurs points de coordonnées ajoutés différemment d'autres points de coordonnées du nuage de points généré à partir des données de capteur de position.

4. Système selon la revendication 1 dans lequel la portion du nuage de points inclut uniquement les un ou plusieurs points de coordonnées ajoutés.

5. Système selon la revendication 1 dans lequel les opérations comprennent en outre la détermination d'une transformation pour aligner une image des une ou plusieurs images des premières données d'image avec l'anatomie de patient correspondante de la région anatomique dans la portion des deuxièmes données d'image, et dans lequel la mise à jour du calage inclut la mise à jour du calage sur la base, au moins en partie, de la transformation.

6. Système selon la revendication 1 dans lequel les opérations comprennent en outre la détermination, sur la base, au moins en partie, des premières données d'image, d'au moins une portion d'un trajet emprunté par le dispositif médical à travers la région anatomique, et dans lequel la génération du calage inclut la génération du calage entre au moins la portion du nuage de points et une section de la région anatomique correspondant à la portion du trajet.

7. Système selon la revendication 1 dans lequel les opérations comprennent en outre l'estimation d'une erreur de calage entre une correspondance des une ou plusieurs correspondant et le calage généré.

8. Système selon la revendication 7 dans lequel les opérations comprennent en outre la mise en couleur d'un affichage du calage généré sur la base, au moins en partie, d'une grandeur de l'erreur de calage estimée.

9. Système selon l'une quelconque des revendications 1 à 4 et 6 à 8, dans lequel les opérations comprennent en outre :
l'informatisation, sur la base, au moins en partie, du calage généré, d'une image virtuelle de l'anatomie de patient de la région anatomique à partir d'une perspective du dispositif de capture d'image à un emplacement actuel du dispositif de capture d'image au sein de la région anatomique ; et
la détermination d'une transformation pour aligner l'image virtuelle avec une image des premières données d'image correspondant à l'emplacement actuel du dispositif de capture d'image.

10. Système selon la revendication 9 dans lequel la mise à jour du calage inclut la mise à jour du calage sur la base, au moins en partie, de la transformation déterminée.

11. Système selon la revendication 9 dans lequel la détermination de la transformation inclut :
la détermination de la transformation pour uniquement une portion du calage généré au sein d'une distance seuil de l'emplacement actuel du dispositif de capture d'image (247) ; ou
la détermination de la transformation pour une phase respiratoire et/ou cardiaque spécifique du patient.

12. Système selon l'une quelconque des revendications 1 à 4 et 6 à 8, dans lequel les opérations comprennent en outre :
l'informatisation, sur la base, au moins en partie, du calage généré, d'une image virtuelle de l'anatomie de patient de la région anatomique à partir d'une perspective du dispositif de capture d'image (247) à un emplacement actuel ou précédent du dispositif de capture d'image (247) au sein de la région anatomique, dans lequel l'image virtuelle est associée à une première estampille temporelle ; et
la détermination d'une image des premières données d'image qui concorde au mieux avec l'image virtuelle, dans lequel l'image des premières données d'image est incluse dans un groupe d'au moins deux images des premières données d'image, et dans lequel chaque image des au moins deux images est associée à une estampille temporelle présente dans une période de temps spécifiée avant, pendant, et/ou après la première estampille temporelle.

13. Système selon la revendication 12 dans lequel les opérations comprennent en outre la détermination d'une différence entre (i) une estampille temporelle associée à l'image des premières données d'image qui concorde au mieux avec l'image virtuelle et (ii) la première estampille temporelle, et dans lequel la mise à jour du calage inclut la mise à jour du calage sur la base, au moins en partie, de la différence déterminée.
